(19) Europäisches Patentamt / European Patent Office / Office européen des brevets

(11) **EP 2 892 642 B1**

(12) **EUROPÄISCHE PATENTSCHRIFT**

(45) Veröffentlichungstag und Bekanntmachung des Hinweises auf die Patenterteilung:
**16.11.2016 Patentblatt 2016/46**

(21) Anmeldenummer: **13771075.2**

(22) Anmeldetag: **05.09.2013**

(51) Int Cl.:
*B01J 13/00* ^(2006.01)

(86) Internationale Anmeldenummer:
**PCT/EP2013/068343**

(87) Internationale Veröffentlichungsnummer:
**WO 2014/037429 (13.03.2014 Gazette 2014/11)**

(54) **VERFAHREN ZUR HERSTELLUNG EINES POLYGLYCERIN-NANOGELS ZUR VERKAPSELUNG UND FREISETZUNG BIOLOGISCH AKTIVER SUBSTANZEN**

METHOD FOR PRODUCING A POLYGLYCERIN NANOGEL FOR ENCAPSULATION AND RELEASE OF BIOLOGICALLY ACTIVE SUBSTANCES

PROCÉDÉ DE PRÉPARATION D'UN NANOGEL DE POLYGLYCÉROL POUR L'ENCAPSULAGE ET LA LIBÉRATION DE SUBSTANCES BIOLOGIQUEMENT ACTIVES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.09.2012 DE 102012108345**

(43) Veröffentlichungstag der Anmeldung:
**15.07.2015 Patentblatt 2015/29**

(73) Patentinhaber: **Freie Universität Berlin**
**14195 Berlin (DE)**

(72) Erfinder:
- **HAAG, Rainer**
  **12209 Berlin (DE)**
- **STEINHILBER, Dirk**
  **Quincy, MA 02171 (US)**
- **FRIESS, Wolfgang**
  **82393 Iffeldorf (DE)**
- **Hedtrich, Sarah**
  **10117 Berlin (DE)**
- **WITTING, Madeleine**
  **81241 München (DE)**

(74) Vertreter: **Maikowski & Ninnemann**
**Patentanwälte Partnerschaft mbB**
**Postfach 15 09 20**
**10671 Berlin (DE)**

(56) Entgegenhaltungen:
**EP-A1- 2 138 527    US-A1- 2009 117 070**

- **MALKOCH MICHAEL ET AL: "Synthesis of well-defined hydrogel networks using click chemistry", CHEMICAL COMMUNICATIONS; [6015D], ROYAL SOCIETY OF CHEMISTRY, GB, Nr. 26, 14. Juli 2006 (2006-07-14), Seiten 2774-2776, XP002462132, ISSN: 1359-7345, DOI: 10.1039/B603438A**
- **ALEXANDER V. KABANOV ET AL: "Nanogels as Pharmaceutical Carriers: Finite Networks of Infinite Capabilities", ANGEWANDTE CHEMIE INTERNATIONAL EDITION, Bd. 48, Nr. 30, 13. Juli 2009 (2009-07-13) , Seiten 5418-5429, XP055101122, ISSN: 1433-7851, DOI: 10.1002/anie.200900441**

**Beschreibung**

Die vorliegende Erfindung betrifft ein Verfahren zur Herstellung eines Polyglycerin-Nanogels gemäß dem Oberbegriff des Anspruchs 1.

Aus dem Stand der Technik sind verschiedene Verfahren zur Herstellung von Polyglycerin-Nanopartikeln oder Polyglycerin-Nanogelen bekannt (A. L. Sisson, I. Papp, K. Landfester, R. Haag, Macromolecules 2009 42, 556-559; A. L. Sisson, D. Steinhilber, T. Rossow, P. Welker, K. Licha, R. Haag, Ang. Chem. Int. Ed. 2009 48, 7540-7545; D. Steinhilber, A. L. Sisson, D. Mangoldt, P. Welker, K. Licha, R. Haag, Adv. Funct. Mater. 2010 20, 4133-4138; H. Zhou, D. Steinhilber, H. Schlaad, A. L. Sisson, R. Haag, React. Funct, Polym. 2011, 71, 356-361).

Auch beschreibt beispielsweise die EP 2 138 527 A1 ein Verfahren zur Herstellung von Polyglycerin-Nanopartikeln. Gemäß diesem Verfahren des Stands der Technik werden Mini-oder Mikroemulsionen gebildet, um Nanopartikel herzustellen. Bei dem Verfahren wird stets unter Einsatz einer oberflächenaktiven Substanz gearbeitet. Zudem erfolgt an verschiedenen Stufen des Verfahrens eine Ultraschallbehandlung. Auch sind die Reaktionstemperaturen mitunter recht hoch. Unter derart harschen Bedingungen ist es nicht möglich, Substanzen, die chemisch oder physikalisch empfindlich gegenüber Temperatur, Grenzflächeneffekten, Ultraschall und extremen pH-Werten sind, wie etwa Proteine, unter Erhalt der Aktivität in die derart gebildeten Polyglycerin-Nanogele einzukapseln.

Aus der US 2009/0011420 A1 sind polymere Nanogele bekannt, die mittels radikalischer Vernetzung gebildet werden. Eine solche radikalische Vernetzung ist mit einer Einbettung labiler Biomoleküle in ein derartiges Nanogel inkompatibel; die Radikale schädigen die labilen Biomoleküle nachhaltig und können sie zudem inaktivieren (Pattison, David I., Aldwin Suryo Rahmanto, and Michael J. Davies. "Photo-oxidation of proteins." Photochemical & Photobiological Sciences 11.1 (2012): 38-53.). Die in dieser US-Patentanmeldung beschriebenen Nanogele eignen sich daher nicht zur Einbettung labiler Biomoleküle, sondern zur elektrophoretischen Trennung von DNA-Molekülen nach erfolgter Ausbildung der Nanogele.

Aus der DE 10 2008 030 992 A1 sind Polyglycerinverbindungen bekannt, die aus einem rein hydrophoben Kern bestehen, was die Verkapslung hydrophiler Substanzen praktisch ausschließt. Darüber hinaus ist durch das Verkapseln in wässriger Phase grundsätzlich nur eine geringe Beladungseffizienz mit hydrophilen Substanzen zu erwarten. Damit wird in jener Patentanmeldung kein geeignetes Verfahren beschrieben, mit dem labile Biomoleküle effektiv in eine Polyglycerinverbindung eingebettet werden können.

Der vorliegenden Erfindung liegt die Aufgabe zugrunde, ein neuartiges Herstellungsverfahren für ein Polyglycerin-Nanogel anzugeben, bei dem labile Substanzen wie etwa therapeutische Enzyme oder Proteine während der Bildung des Nanogels in das Nanogel unter Erhalt der Aktivität eingebettet werden können.

Diese Aufgabe wird mit einem Verfahren mit den Merkmalen des Anspruchs 1 gelöst. Bei diesem Verfahren wird zunächst eine wässrige Lösung erster Polyglycerin-Makromonomere mit einer wässrigen Lösung zweiter Polyglycerin-Makromonomere gemischt. Die ersten Polyglycerin-Makromonomere sind mit einer ersten reaktiven Gruppe modifiziert und die zweiten Polyglycerin-Makromonomere sind mit einer zweiten reaktiven Gruppe modifiziert. Die erste reaktive Gruppe und die zweite reaktive Gruppe können unter Ausbildung einer chemischen (kovalenten) Bindung miteinander reagieren. Eine solche Reaktion findet jedoch nur statt, wenn die Konzentration der beiden Makromonomere ausreichend hoch ist. Bei niedriger Konzentration der Makromomonomere kommt es noch nicht zu einer Reaktion. Anschließend wird die Mischung aus den ersten Polyglycerin-Makromonomeren und den zweiten Polyglycerin-Makromonomeren in ein organisches Nichtlösungsmittel bzw. eine organische Flüssigkeit überführt. Dadurch steigt die Konzentration der Makomonomere schlagartig an, da sie sich in dem Nichtlösungsmittel nicht lösen. Nun kommt es zu einer Nanoausfällung eines Polyglycerin-Nanogels, das aus den ersten Polyglycerin-Makromonomeren und den zweiten Polyglycerin-Makromonomeren besteht, wobei die ersten Makromonomere kovalent mit den zweiten Makromonomeren verbunden sind, um das Nanogel zu bilden.Die kovalente Bindung zwischen den ersten Polyglycerin-Makromonomeren und den zweiten Polyglycerin-Makromonomeren wird dabei durch eine Reaktion der ersten reaktiven Gruppe mit der zweiten reaktiven Gruppe ausgebildet, die erst infolge der Überführung der Mischung in das organische Nichtlösungsmittel spontan abläuft. Das erfindungsgemäß beanspruchte Verfahren zeichnet sich dadurch aus, dass das organische Nichtlösungsmittel bzw. die organische Flüssigkeit mit Wasser mischbar ist und dass das Verfahren gänzlich ohne Zugabe oberflächenaktiver Substanzen durchgeführt wird.

Mit anderen Worten ausgedrückt, sind für die chemische Reaktion (also die Ausbildung der kovalenten Bindung zwischen den ersten Makromolekülen und den zweiten Makromolekülen) und die damit einhergehende Nanopartikelbildung keine Tenside oder amphiphilen Substanzen, welche Assoziationskolloide bilden und die Grenzflächenspannung herabsetzen, notwendig. Insbesondere durch den Verzicht auf oberflächenaktive Substanzen wie etwa Tenside ist es möglich, Proteine wie etwa therapeutische Enzyme oder andere biologisch aktive Substanzen zusammen mit den Makromonomeren zu einem Polyglycerin-Nanogel auszufällen. Dies ist mit den aus dem Stand der Technik bekannten Verfahren nicht möglich, da es hier aufgrund der harschen Bedingungen (insbesondere durch harsche Ultraschallbehandlung und die Anwesenheit oberflächenaktiver Substanzen) regelmäßig zur Denaturierung von Proteinen oder Beeinflussung anderer aktiver Substanzen kommt.

Überraschenderweise konnten die Erfinder jedoch zeigen, dass die Anwesenheit von oberflächenaktiven Substanzen nicht erforderlich ist, wenn in einem mit Wasser mischbaren organischen Nichtlösungsmittel gearbeitet wird. Denn wenn die Mischung aus ersten Polyglycerin-Makromonomeren und zweiten Polyglycerin-Makromonomeren in das organische Nichtlösungsmittel überführt wird, kommt es zur Diffusion von Wasser aus einzelnen Wassertröpfchen, die die Makromonomere enthalten, in das umgebende Nichtlösungsmittel. Dadurch steigt die Konzentration der Makromonomere in der wässrigen Phase an, wodurch eine kritische Grenzkonzentration überschritten wird. Dadurch wird die Reaktion zwischen ersten Polyglycerin-Makromonomeren und zweiten Polyglycerin-Makromonomeren initiiert.

Geeignete Nichtlösungsmittel bzw. organische Flüssigkeiten sind z.B. polare organische Flüssigkeiten, die mit Wasser mischbar sind, jedoch Polyglycerin nicht lösen. Besonders geeignet sind Aceton und Acetonitril.

Bei der beanspruchten Reaktion wird insbesondere kein Tensid gebildet. Bei den Reaktionsprodukten handelt es sich vielmehr um rein hydrophile Strukturen ohne amphiphile und oberflächenaktive Eigenschaften. Im Falle einer Tensidbildung wäre zu erwarten, das nach einer Degradation der gebildeten Nanogele aufgrund spontaner Mizellbildungen nanopartikuläre Strukturen entstehen bzw. bestehen bleiben, welche beispielsweise mit dynamischen Lichtstreuungsmethoden (DLS) nachweisbar wären. Wie die Erfinder zeigen konnten, sind jedoch keinerlei mizellare Strukturen nach einer Nanogel-Degradation nachweisbar

Bei den reaktiven Gruppen handelt es sich vorzugsweise jeweils um bioorthogonale Strukturen, wodurch definitionsgemäß chemische Gruppen beschrieben werden, die lediglich untereinander, aber keinesfalls mit nativen biochemischen Prozessen interagieren oder mit nativen Molekülen reagieren. Eine entsprechend vertiefte und detaillierte Beschreibung derartiger bioorthogonaler Strukturen ist durch C. Bertozzi et al. publiziert (C. Bertozzi et al. Angew. Chem. Int. Ed. Engl. 2009, 48, 6974-6998).

Das hier beanspruchte Nanopräzipitationsverfahren kommt zudem bevorzugt ohne Ultraschallbehandlung aus. Das Verfahren kann daher in einer Variante explizit ohne Ultraschallbehandlung durchgeführt werden. Dies ermöglicht insgesamt sehr schonende Reaktionsbedingungen, durch die Enzyme, andere Proteine oder andere aktive Substanzen nicht angegriffen werden.

Aber auch ohne den zusätzlichen Einsatz von Enzymen, anderen Proteinen oder anderen aktiven Substanzen ist dieses Verfahren aufgrund seiner besonders einfachen Durchführung hervorragend zur Herstellung von Polyglycerin-Nanogelen geeignet. In diesem Zusammenhang wird darauf verwiesen, dass der Begriff "Nanogel" vorliegend als Äquivalent zu "Nanopartikel" verwendet wird, da sich dies in der Fachsprache durchgesetzt hat.

Im Gegensatz zu den aus dem Stand der Technik bekannten Verfahren, insbesondere im Gegensatz zu dem in EP 2 138 527 A1 beschriebenen Verfahren, sind/ist

(1) oberflächenaktive Substanzen für die Reaktion gemäß dem vorliegend beanspruchten Verfahren nicht notwendig,

(2) keine Ultraschallbehandlung für die Partikelherstellung notwendig.

Tenside und insbesondere die Ultraschallbehandlung sind Verfahren, welche labile Substanzen wie z.B. Proteine/Peptide maßgeblich schädigen können, wodurch ein Aktivitätsverlust eintreten kann. Durch den mechanischen Stress einer Ultraschallbehandlung können sich innerhalb kürzester Zeit Aggregate bilden, welche neben einem Funktionsverlust auch mit einer Erhöhung der Immunogenität einhergehen können. Das hier beschriebene Verfahren ist wesentlich schonender und ermöglicht erstmals die effiziente und sichere Verkapslung labiler, hydrophiler Substanzen.

Das vorliegend beanspruchte Verfahren macht vorzugsweise von einem spontanen Konzentrationsgradienten in der wässrigen Phase Gebrauch, der sich automatisch durch eine Wasserdiffusion aus der wässrigen Phase heraus in die organische Phase bei der Durchführung des Verfahrens einstellt. Das heißt, das Verfahren läuft vorzugsweise spontan ab. Genauer ausgedrückt, läuft das Ausfällen des Polyglycerin-Nanogels nach Überführen der Mischung in das organische Lösungsmittel vorzugsweise spontan ab. Eine derartige spontane Reaktion ist dem Fachmann grundsätzlich bekannt. So wird eine spontane Reaktion in den "Prinzipien der Chemie" von Dickerson et al. (2. Auflage 1988, Walter de Gruyter Verlag, Berlin, New York) in Kapitel 5-1 auf Seite 187 als eine chemische Reaktion definiert, die, wenn ihr genug Zeit gelassen wird, von selbst ablaufen wird.

Durch eine aktive Vereinigung der beiden Polyglycerin-Makromonomer-Lösungen in dem Nichtlösungsmittel durch einen Operator wird folglich vorzugsweise die spontane Ausfällung und Vernetzung des Nanogels initiiert. Somit liegt in diesem Fall eine doppelte Spontanität hinsichtlich der Nanopartikelbildung und der chemischen Vernetzung vor.

In einer bevorzugten Ausgestaltung des Verfahrens wird mit sehr niedriger Ausgangskonzentration der als Edukte eingesetzten Makromonomere gearbeitet. Denn auf diese Weise wird sichergestellt, dass die chemische Reaktion zwischen den ersten Polyglycerin-Makromonomeren und den zweiten Polyglycerin-Makromonomeren in der wässrigen Phase noch nicht beginnt, sondern erst durch eine Aufkonzentration der Makromonomere initiiert wird. Vorzugsweise liegen die ersten Makromonomere daher in einer ersten Konzentration und die zweiten Makromonomere in einer zweiten Konzentration vor, wobei die erste und die zweite Konzentration unabhängig voneinander im Bereich von 0,1 bis 30 mg/ml liegen. Ein bevorzugter Konzentrationsbereich ist 0,2 bis 25 mg/ml, insbesondere 0,5 bis 20 mg/ml, insbesondere 1 bis 15 mg/ml, insbesondere 2 bis 12 mg/ml und ganz besonders 3 bis 10 mg/ml. Die erste Konzentration und die zweite Konzentration können dabei gleich oder verschieden sein.

Durch die Wahl der Konzentration der Makromonomere lässt sich die Größe des erhaltenen Nanogels einstellen. So konnte gezeigt werden, dass der Durchmesser des gebildeten Nanogels in Abhängigkeit der Konzentration der Makromonomere im Bereich von etwa 50 nm bis etwa 1.000 nm variiert werden kann. Dabei sind insbesondere Nanogele mit einem Durchmesser (gemessen in Aceton und bestimmt mittels dynamischer Lichtstreuung) von 100 bis 600 nm, insbesondere 200 bis 500 nm und ganz besonders von 300 bis 400 nm geeignete Varianten.

In einer alternativen Ausgestaltung des Verfahrens wird das Polyglycerin-Nanogel nach dem Ausfällen in eine wässrige Phase überführt. Dadurch kommt es zum Eindringen von Wasser in das Nanogel, wodurch das gebildete Polyglycerin-Nanogel quillt. Dies lässt sich durch eine Vergrößerung des Durchmessers des Nanogels beobachten. Ein derart gequollenes Nanogel lässt sich in besonders vorteilhafter Weise als Trägersubstanz für Proteine und andere aktive Substanzen verwenden.

Wie bereits mehrfach angedeutet, wird das Verfahren in einer bevorzugten Ausgestaltung derart ausgeführt, dass das Ausfällen des Nanogels in Anwesenheit einer labilen Substanz, insbesondere eines Peptids, eines Proteins, eines Enzyms, einer Nukleinsäure und/oder eines Hormons durchgeführt wird. Andere biologisch aktive Substanzen sind ebenfalls als mögliche Ausfällungspartner denkbar. Die Ausfällung des Nanogels in Anwesenheit einer solchen Substanz hat zur Folge, dass diese Substanz im Inneren des Nanogels eingebettet wird. Polyglycerine zeigen bekannterweise geringe Wechselwirkungen mit Proteinen, wodurch matrixinduzierte Proteindenaturierung verhindert werden kann. Das Verwenden von verzweigten Polyglycerinen erzeugt ein engmaschiges Nanogel Netzwerk, in welchem die Proteine durch gehinderte Diffusion stabil verkapselt werden. Solange das Nanogel intakt bleibt, verbleibt auch das Protein bzw. die andere aktive Substanz im Inneren des Nanogels.

In einer weiteren Ausgestaltung des Verfahrens weisen die ersten Polyglycerin-Makromonomere und/oder die zweiten Polyglycerin-Makromonomere eine pH-labile Gruppe auf. Diese pH-labile Gruppe ist auch nach Reaktion der ersten reaktiven Gruppe der ersten Makromonomere in dem gebildeten Nanogel enthalten. Die pH-labile Gruppe kann nun dazu verwendet werden, die Struktur des gebildeten Nanogels in Abhängigkeit des pH-Wertes aufzulösen. Auf diese Weise wird also ein bioabbaubares Polyglycerin-Nanogel bereitgestellt. Wenn die pH-labile Gruppe infolge einer pH-Wert-Änderung gespalten wird, kommt es zum Aufbrechen des Polyglycerin-Nanogels und damit zur Freisetzung einer in dem Nanogel enthaltenen aktiven Substanz, wie etwa eines Proteins. Die Reste der Polyglycerinstruktur können dann von einem Organismus ohne weiteres abgebaut werden, da sie nicht toxisch sind.

Der Begriff "pH-labil" definiert folglich die Eigenschaft, dass bei bestimmten pH-Werten (in Abhängigkeit von der Bindung der reaktiven Gruppen) die zuvor ausgebildeten kovalenten Bindungen aufgespalten werden und es dadurch zu einer Desintegration der Nanogele kommt. Reaktive Gruppen unterscheiden sich von den pH-labilen Gruppen durch ihre pH-unabhängige reaktive Stabilität. Es ist möglich, dass die pH-labilen Gruppen kovalente Bindungen ausbilden. Diese kovalenten Bindungen sind dann - anders als bei kovalenten Bindungen, die durch nicht pH-labile Gruppen gebildet werden - gewünscht pH-labil, also in Abhängigkeit des pH-Werts spaltbar.

In einer bevorzugten Ausgestaltung ist die pH-labile Gruppe ausgewählt aus der Gruppe bestehend aus Acetalen, Ketalen, Enolethern, Enolestern, Amiden von 2,3-disubstituierten Maleinsäurederivaten, Iminen, Imminiumverbindungen, Enaminen, Silylethern und Silylenolethern.

Dabei haben sich insbesondere Acetale als geeignete pH-labile Gruppen herausgestellt. Alkylacetale oder Arylacetale, wie etwa das Benzacetal, haben sich als besonders geeignete pH-labile Gruppe herausgestellt.

Eine besonders vorteilhafte und einfache Synthese des Polyglycerin-Nanogels ist möglich, wenn die pH-labile Gruppe mit der reaktiven Gruppe verknüpft ist und gemeinsam an das Polyglyceringrundgerüst angehängt wird. Daher weisen die ersten Polyglycerin-Makromonomere und/oder die zweiten Polyglycerin-Makromonomere vorzugsweise eine endständige Modifizierung des Typs -R-R' auf, die kovalent mit einem linearen oder verzweigten Polyglyceringerüst der ersten Polyglycerin-Makromonomere und/oder der zweiten Polyglycerin-Makromonomere verbunden ist. Das Polyglyceringrundgerüst kann dabei auch als "A" bezeichnet werden, wodurch sich eine schematische Struktur der Form A-R-R' ergibt.

R bedeutet dabei eine pH-labile Gruppe und R' eine endständige Gruppe, die eine Reaktion gemäß der Klick-Chemie eingehen kann. Vorzugsweise ist R' eine bioorthogonale Gruppe, welche dann mit den bioorthogonalen reaktiven Gruppen des jeweils anderen Makromonomers bei Zusammenführen in dem Nichtlösungsmittel mittels Klick-Chemie reagieren und eine kovalente Bindung ausbilden. Der dem Fachmann allgemein bekannte Begriff der Klick-Chemie beschreibt chemische Reaktionen, die bioorthogonal, chemoselektiv und in hoher Geschwindigkeit bei annähernd 100 % Umsatz durchgeführt werden können. Reaktionen gemäß der Klick-Chemie sind modular ausgestaltet und weisen eine breite Anwendungsmöglichkeit, hohe Ausbeuten, und eine hohe thermodynamische Antriebskraft auf. Zudem werden praktisch keine Nebenprodukte gebildet. Sie sind beispielsweise von Sharpless et al. (K. B. Sharpless et al. Angew. Chem. Int. Ed. Engl. 2001, 40, 2004-2021) beschrieben. Dabei können Reaktionen gemäß der Klick-Chemie beispielsweise durch Kupfer katalysiert oder aber unkatalysiert ablaufen.

Die durch die pH-labilen Gruppen R eingeführten pH-labilen Bindungen weisen vorzugsweise eine hohe Stabilität bei einem pH-Wert von 7-8, insbesondere bei einem pH-Wert von 7,2 bis 7,8, insbesondere bei einem pH-Wert von 7,4 bis 7,6 auf, also unter den üblicherweise gewählten Reaktionsbedingungen. Werden allerdings saure pH-Werte erreicht (pH 4-6, insbesondere pH 4,5-5,5, insbesondere pH 4,8-5,3), spaltet sich die pH-labile Bindung und das Polymernetzwerk wird abgebaut.

Vorzugsweise ist nur in einem Typ Makromonomere eine pH-labile Gruppe eingebaut. Durch eine anschließende Verknüpfung des ersten Typs Makromonomere mit dem zweiten Typ Makromonomere ist eine entsprechend homogene Verteilung von pH-labilen Gruppen über das gesamte Polyglycerin-Nanogel gewährleistet.

Insbesondere dann, wenn zusätzliche Substanzen in das Nanogel mit eingebettet werden sollen, sind besonders milde Reaktionsbedingungen von Vorteil. Das vorliegende Verfahren wird daher vorzugsweise bei einer Temperatur von 0 °C bis 25 °C durchgeführt. Als besonders geeignete Reaktionsbedingungen haben sich Temperaturen von 2 °C bis 20 °C, insbesondere von 3 °C bis 15 °C und ganz besonders von 4 °C bis 10 °C herausgestellt. Andere bekannte Verfahren zur Herstellung von Polyglycerin-Nanogelen laufen bei derart niedrigen Temperaturen nicht in hinreichend hoher Reaktionsgeschwindigkeit ab.

Die zuvor bereits erwähnte Klick-Chemie kann in besonders vorteilhafter Weise durchgeführt werden, wenn eine Reaktion zwischen einem Alkin und einem Azid erfolgt. Daher handelt es sich bei der ersten reaktiven Gruppe vorzugsweise um eine Alkingruppe und bei der zweiten reaktiven Gruppe um eine Azidgruppe (oder umgekehrt). Die erste reaktive Gruppe bzw. die zweite reaktive Gruppe entspricht der Gruppe R' einer der zuvor erläuterten alternativen Ausführungsformen des Verfahrens. Als besonders geeignete Alkingruppen haben sich solche Alkingruppen mit einem, zwei, drei, vier, fünf, sechs, sieben, acht, neun oder zehn Kohlenstoffatome herausgestellt, wobei Linker mit einer endständigen Dreifachbindung gemäß $-(CH_2)_n-\equiv$ mit n= 1-10 oder gespannte zyklische Alkine einsetzbar sind. Diese Gruppen können dabei über Heteroatome, wie etwa Sauerstoff-, Stickstoff- oder Schwefelatome mit weiteren Modifikationsgruppen bzw. mit dem Kern des Polyglycerin-Makromonomers verbunden sein. Eine besonders geeignete Alkingruppe ist die Propargyloxy-Gruppe.

Um sicherzustellen, dass die Proteine bzw. anderen aktiven Substanzen selbst nicht an der Vernetzungsreaktion zwischen dem ersten Makromonomer und dem zweiten Makromonomer teilnehmen und um somit eine mögliche Denaturierung der Proteine bzw. anderen aktiven Substanzen zu verhindern, wird das Verfahren vorzugsweise als bioorthogonale Reaktion ausgeführt. Ein besonders geeigneter Reaktionsmechanismus besteht in der Kupferkatalysierten 2,3-Cycloaddition multifunktioneller Polyglycerin-Alkine und Polyglycerin-Azide. Um Kupferkontaminationen zu umgehen, eignet sich aber auch eine katalysatorfreie Vernetzungsreaktion von gespannten Polyglycerin-Cyclooctinen mit Polyglycerin-Aziden. Weitere Reaktionen sind ebenso denkbar.

In einer Variante erfolgt das Ausfällen des Polyglycerin-Nanogels ohne Zugabe einer kupferhaltigen Verbindung. Das heißt, die Reaktionsumgebung, in der die Ausfällungsreaktion erfolgt, ist vorzugsweise vollständig kupferfrei. Insbesondere wird vorzugsweise im gesamten Verfahren keine kupferhaltige Verbindung eingesetzt. Überraschenderweise konnte gezeigt werden, dass ein Polyglycerin-Nanogel auch ohne Einsatz des toxischen Agens Kupfer effektiv ausgefällt werden kann, wobei eine Einbettung biologisch aktiver Substanzen auch in diesem Fall möglich ist. Diese Variante ermöglicht ein noch schonenderes Einbetten biologischer aktiver Substanzen.

Beschrieben wird auch ein Polyglycerin-Nanogel, das mindestens eine pH-labile Gruppe aufweist. Dabei können in einer bevorzugten Ausgestaltung des Polyglycerin-Nanogels die im Zusammenhang mit der Erläuterung der möglichen Ausgestaltungen des Verfahrens zur Herstellung eines Polyglycerin-Nanogels erläuterten pH-labilen Gruppen eingesetzt werden. Darüber hinaus sind auch die weiteren alternativen Verfahrensausgestaltungen in analoger Weise auf das beanspruchte Polyglycerin-Nanogel übertragbar.

Beschrieben wird zudem die Verwendung eines solchen Polyglycerin-Nanogels als Träger für eine Substanz, beispielsweise für ein Peptid, ein Protein, eine Nukleinsäure und/oder ein Hormon. Weitere Vorteile und Einzelheiten der vorliegenden Erfindung werden anhand von Abbildungen und Ausführungsbeispielen näher erläutert. Es zeigen:

Fig. 1       eine schematische Darstellung eines Ausführungsbeispiels eines Verfahrens zur Herstellung eines Polyglycerin-Nanogels;

Fig. 2A       eine grafische Darstellung der Zersetzung eines pH-labilen Polyglycerin-Nanogels bei unterschiedlichen pH-Werten;

Fig. 2B       eine grafische Darstellung der L-Asparaginase-II-Freisetzung aus einem pH-labilen Polyglycerin-Nanogel bei unterschiedlichen pH-Werten;

Fig. 3       eine schematische Darstellung eines Ausführungsbeispiels eines Verfahrens zur Herstellung eines Polyglycerin-Nanogels mit gleichzeitiger Einbettung eines Proteins;

Fig.4A       zweite Ableitungen von Absorptionsspektren des Modellproteins L-Asparaginase II in der Amid-I-Region nach Verkapslung und Abbau der Nanogele;

Fig.4B zweite Ableitungen von Absorptionsspektren des Modellproteins L-Asparaginase II in der Amid-II-Region nach Verkapslung und Abbau der Nanogele,

Fig. 5 eine grafische Darstellung der Enzymaktivität des Modellproteins L-Asparaginase II nach Verkapslung und Freisetzung aus den Nanogelen und

Fig. 6 ein Flussdiagramm zur Veranschaulichung der einzelnen Verfahrensschritte eines Ausführungsbeispiels.

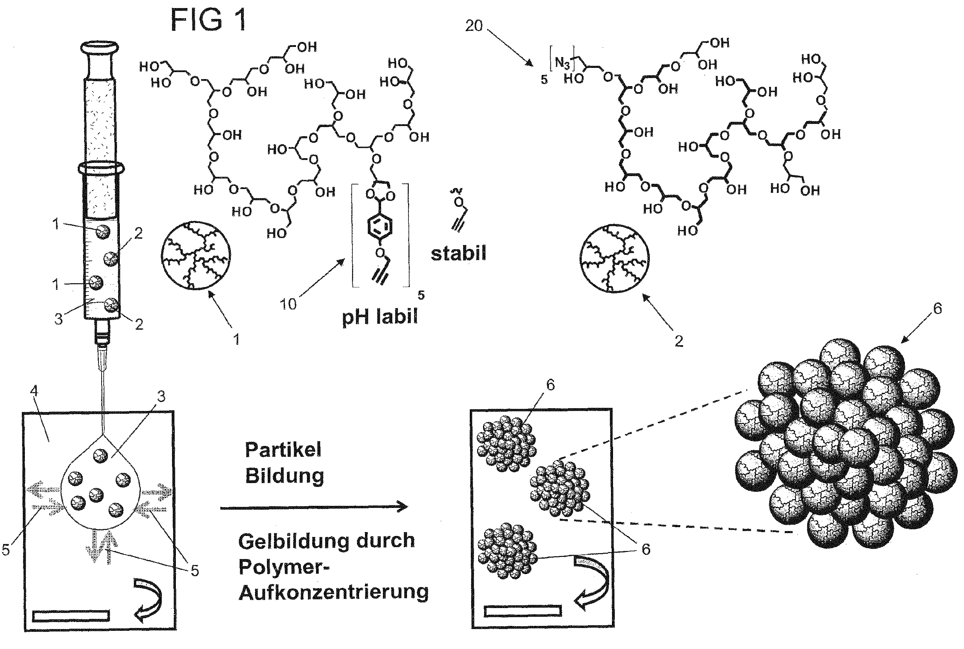

Die Figur 1 zeigt eine schematische Darstellung eines Verfahrens zur Herstellung eines Polyglycerin-Nanogels. Erste Polyglycerin-Makromonomere (1) werden dabei mit zweiten Polyglycerin-Makromonomeren (2) in niedriger Konzentration miteinander vermischt. Die ersten Makromonomere tragen jeweils fünf Propargyloxybenzacetal-Gruppen (10) als erste reaktive Gruppe bzw. Alkin-Gruppe. Die zweiten Makromonomere tragen jeweils fünf Azid-Gruppen (20) als zweite reaktive Gruppen bzw. Azid-Gruppen. Dabei ist die Alkin-Einheit der Propargyloxybenzacetal-Gruppen (10) endständig angeordnet. Diese Alkin-Einheit ist für sich genommen stabil. Die Azid-Gruppen (20) der zweiten Makromonomere (2) sind ebenfalls endständig angeordnet.

Die Mischung aus ersten Makromonomeren (1) und zweiten Makromonomeren (2) liegt in einer wässrigen Phase vor und wird in Aceton (4) als organisches Nichtlösungsmittel eingebracht. Nachfolgend kommt es zu einer Diffusion (5) des Wassers aus der wässrigen Phase (3) in das Aceton (4). Dadurch erhöht sich die Konzentration der ersten Makromonomere (1) und der zweiten Makromonomere (2) in der wässrigen Phase (3). Dadurch wird die zur Reaktionsinitiierung kritische Konzentration der Makromonomere (1, 2) erreicht, so dass es zur Reaktion zwischen den Alkin-Gruppen (10) und den Azid-Gruppen (20) kommt. Es bilden sich kovalente Bindungen zwischen den ersten Makromonomeren (1) und den zweiten Makromonomeren (2) aus, die in einer Bildung von Nanopartikeln (6) resultiert. Durch Quellen bildet sich auf diese Weise ein Polyglycerin-Nanogel 6. Diese in der Figur 1 schematisch dargestellte Reaktion wird nun anhand eines konkreten Ausführungsbeispiels näher dargestellt.

### Herstellung von p-Propargyloxy-Benzaldehyd

2,00 g 4-Hydroxybenzaldehyd (16,38 mmol) wurden in 50 ml Aceton gelöst. 15,15 g Kaliumcarbonat (109,62 mmol) wurden hinzugefügt, und die Suspension wurde für 30 Minuten unter Rückfluss gerührt. Nachdem die Lösung auf Raumtemperatur abgekühlt war, wurden 2,12 ml Propargylbromid (19,10 mmol) während eines Zeitraums von 2,5 Stunden hinzugegeben. Anschließend wurde die Suspension unter Rückfluss für 1,5 Stunden erwärmt. Dann wurde die Suspension filtriert und das Lösungsmittel des Filtrats im Vakuum abgedampft. Es wurden 50 ml Dichlormethan hinzugegeben und die organische Phase anschließend zweimal mit 20 ml 1 M Natronlauge und einmal mit 20 ml Wasser gewaschen. Die organische Phase wurde über Magnesiumsulfat getrocknet. Das Rohprodukt wurde mittels Kugelrohrdestillation gereinigt. Es wurde ein weißer kristalliner Feststoff erhalten. Wie eine Analyse ergeben hat, fand eine vollständige Umsetzung bei einer Ausbeute von rund 80 % statt.

### Herstellung von p-Propargyloxy-Benzdimethylacetal

1 g p-Propargyloxy-Benzaldehyd (6,29 mmol) wurden in 10 ml Trimethylorthoformiat gelöst. Anschließend wurden 108 mg trockene p-Toluolsulfonsäure (0,629 mmol) hinzugefügt. Der Reaktionsansatz wurde auf 65 °C für eine Stunde erwärmt und anschließend durch die Zugabe von 25 ml gesättigter Natriumhydrogencarbonatlösung gequencht. Die Mischung wurde drei Mal mit jeweils 25 ml Ethylacetat extrahiert, und die Fraktionen wurden miteinander vereint und über Natriumsulfat getrocknet. Das Ethylacetat wurde abgedampft und p-Propargyloxy-Benzdimethylacetal wurde als gelbliches Öl in einer Ausbeute von 90 % erhalten.

### Herstellung von 3-Azidopropyl 4-Toluolsulfonat

In einem Zweihalskolben, welcher mit einem Tropftrichter und einem Rührfisch ausgestattet ist, wurden 3-Azidopropanol (3.187 g, 31,52 mmol) und Triethylamin (9.65 ml, 69,3 mmol, 2.2 molare Äquivalente (Eq.)) in Dichlormethan (30 ml) gelöst. Nach dem Abkühlen auf 4 °C mittels eines wässrigen Eisbades, wurde eine Lösung von Tosylchlorid (6.61 g, 34.7 mmol, 1.1 Eq.) in Dichlormethan (30ml) innerhalb von 10 min hinzugetropft. Die Reaktion wurde weiter 6 h bei Raumtemperatur durchgeführt und der Reaktionsverlauf wurde durch IR-Spektroskopie und Dünnschichtchromatographie verfolgt. Nach dem Abfiltrieren des gebildeten Salzes wurde das Filtrat eingeengt, in Dichlormethan (50 ml) aufgenommen und mit einer 1 M $NH_4Cl$-Lösung (20 ml) gewaschen. Die organische Phase wurde über MgSO4 getrocknet und eingeengt. Die Substanz wurde letztlich durch Säulenchromatographie auf Silikagel aufgereinigt (Hexan/Ethylacetat, 3:1) um 3-Azidopropyl 4-Toluolsulfonat (6.44 g, 80%) als gelbe Flüssigkeit zu erhalten. $R_f$: 0.64 (Hexan/Ethylacetat, 3:1).

**Herstellung von 4-(3-Azidopropoxy)-2-Methoxybenzaldehyd**

In eine Lösung von 4-Hydroxy-2-Methoxybenzaldehyd (3.66 g, 23.9 mmol, 1.2 Eq) und 3-Azidopropyl 4-Toluolsulfonat (5.08 g, 19.91 mmol, 1 Eq.) in Aceton (70 ml) wurde $K_2CO_3$ (16.50 g, 119.5 mmol, 5 Eq.) zugegeben. Die Reaktion wurde 16 h unter Rückfluss durchgeführt und nach Abkühlen auf Raumtemperatur wurde Salz abfiltriert und das Filtrat eingeengt. Nach der Aufnahme des Rückstandes in Dichloromethan (20 ml) wurde die organische Phase mit Wasser gewaschen (20 ml), über $MgSO_4$ getrocknet und dann eingeengt. Die Substanz wurde letztlich durch Säulenchromatographie auf Silikagel aufgereinigt (Hexan/Ethylacetat, 3:1) um 4-(3-Azidopropoxy)-2-Methoxybenzaldehyd (3.98 g, 85%) als transparente Flüssigkeit zu erhalten. $R_f$: 0.54 (Hexan/Ethylacetat, 3:1).

**Herstellung von 4-(3-Azidopropoxy)benzaldehyd**

In eine Lösung von 4-Hydroxybenzaldehyd (2.39 g, 19.61 mmol, 1.2 Eq) und 3-Azidopropyl 4-Toluolsulfonat (5.08 g, 19.91 mmol, 1 Eq.) in Aceton (70 ml) wurde $K_2CO_3$ (13.55 g, 98.05 mmol, 5 Eq.) zugegeben. Die Reaktion wurde 16 h unter Rückfluss durchgeführt und nach Abkühlen auf Raumtemperatur wurde Salz abfiltriert und das Filtrat eingeengt. Nach der Aufnahme des Rückstandes in Dichloromethan (20 ml) wurde die organische Phase mit Wasser gewaschen (20 ml), über $MgSO_4$ getrocknet und dann eingeengt. Die Substanz wurde letztlich durch Säulenchromatographie auf Silikagel aufgereinigt (Hexan/Ethylacetat, 2:1) um 4-(3-Azidopropoxy)benzaldehyd (3.98 g, 91 %) als transparente Flüssigkeit zu erhalten. $R_f$: 0.67 (Hexan/Ethylacetat, 2:1).

**Herstellung von 4-(3-Azidopropoxy)-2-Methoxybenzaldehyd-Dimethylacetal**

In eine Lösung von 4-Azidopropoxy-2-methoxyybenzaldehyd (2.00 g, 9.80 mmol) in entgastem Methanol (20 ml) wurde Trimethylorthoformiat (5.20 ml, 49.02 mmol, 5 Eq.) und wasserfreies PTSA (169 mg, 0.98 mmol) zugegeben. Die Reaktion wurde 20 h unter Rückfluss durchgeführt und nach Abkühlen mit wässrigen Ammoniak (0.5 ml) gequencht. Nach Einengen der Lösungen wurde Ethylacetat (50 ml) zugegeben und die Lösung wurde mit Wasser (50 ml) gewaschen. Nach dem Trocknen der organischen Phase über MgSO4 wurde die organische Phase eingeengt um 4-(3-Azidopropoxy)-2-Methoxybenzaldehyd Dimethylacetal (2.32 g, 95%) als gelbe Flüssigkeit zu erhalten. Rf : 0.84 (Hexan/Ethylacetat, 3:1).

**Herstellung von 4-(3-Azidopropoxy)benzaldehyd Dimethylacetal.**

In eine Lösung von 4-Azidopropoxybenzaldehyd (2.00 g, 8.51 mmol) in entgastem Methanol (20 ml) wurde Trimethylorthoformiat (4.64 ml, 43.72 mmol, 5 Eq.) und wasserfreies PTSA (169 mg, 0.98 mmol) zugegeben. Die Reaktion wurde 20 h unter Rückfluss durchgeführt und nach Abkühlen mit wässrigen Ammoniak (0.5 ml) gequencht. Nach Einengen der Lösungen wurde Ethylacetat (50 ml) zugegeben und die Lösung wurde mit Wasser (50 ml) gewaschen. Nach dem Trocknen der organischen Phase über $MgSO_4$ wurde die organische Phase eingeengt um 4-(3-Azidopropoxy)-2-Methoxybenzaldehyd Dimethylacetal (2.38 g, 97%) als gelbe Flüssigkeit zu erhalten. $R_f$: 0.57 (Hexan/Ethylacetat, 4:1).

**Herstellung von hPG$_{7,7}$, welches mit 7 p-Azidopropoxy-Methoxybenzacetal-Einheiten funktionalisiert ist (hPG$_{7,7}$-7-p-Azidopropoxy-Benzacetal)**

1 g hPG$_{7,7}$ (0,13 mmol) und 365.3 mg von 4-(3-Azidopropoxy)-2-Methoxybenzaldehyd Dimethylacetal (1,3 mmol) wurden in 4 ml n-Methyl-2-Pyrrolidon gelöst, und 22 mg wasserfreie p-Toluolsulfonsäure (0,13 mmol) wurde hinzugefügt. Der Reaktionsansatz wurde für drei Stunden auf Raumtemperatur gehalten und das kondensierte Methanol wurde von dem Reaktionsansatz durch Kryodestillation entfernt. Die Reaktion wurde durch die Zugabe von 1 ml wässrigen Ammoniaks gequencht. Das n-Methyl-2-Pyrrolidon wurde durch Kryodestillation abgedampft, und der verbleibende Rest in basifiziertem Wasser erneut gelöst (basifiziertes Wasser enthält 0,05 Gew.-% wässrigen Ammoniak). Die Lösung wurde im basifizierten Wasser für fünf Tage dialysiert, wobei das Dialysat alle drei Stunden gewechselt wurde. Nach Gefriertrocknung wurde mit sieben p-Azidopropoxy-Methoxybenzacetal Einheiten funktionalisiertes hPG$_{7,7}$ als viskoses Wachs erhalten. Die Umsetzung erfolgte zu 71 % bei einer Ausbeute von 78 %.

**Herstellung von hPG$_{7,7}$, welches mit 7 p-Azidopropoxybenzacetal-Einheiten funktionalisiert ist (hPG$_{7,7}$-7-p-Azidopropoxy-Benzacetal)**

1 g hPG$_{7,7}$ (0,13 mmol) und 326.3 mg von 4-(3-Azidopropoxy)benzaldehyd Dimethylacetal (1,3 mmol) wurden in 4 ml n-Methyl-2-Pyrrolidon gelöst, und 22 mg wasserfreie p-Toluolsulfonsäure (0,13 mmol) wurde hinzugefügt. Der Reaktionsansatz wurde für drei Stunden auf Raumtemperatur gehalten und das kondensierte Methanol wurde von dem Reaktionsansatz durch Kryodestillation entfernt. Die Reaktion wurde durch die Zugabe von 1 ml wässrigen Ammoniaks gequencht. Das n-Methyl-2-Pyrrolidon wurde durch Kryodestillation abgedampft, und der verbleibende Rest in basifiziertem Wasser erneut gelöst (basifiziertes Wasser enthält 0,05 Gew.-% wässrigen Ammoniak). Die Lösung wurde im basifizierten Wasser für fünf Tage dialysiert, wobei das Dialysat alle drei Stunden gewechselt wurde. Nach Gefriertrocknung wurde mit sieben p-Azidopropoxy-Methoxybenzacetal Einheiten funktionalisiertes hPG$_{7,7}$ als viskoses Wachs erhalten. Die Umsetzung erfolgte zu 69 % bei einer Ausbeute von 83 %.

**Herstellung von hPG$_{7,7}$, welches mit 7p-Propargyloxy-Benzacetal-Einheiten funktionalisiert ist (hPG$_{7,7}$-7-p-Propargyloxy-Benzacetal)**

1 g hPG$_{7,7}$ (0,13 mmol) und 250 mg p-Propargyloxy-Benzdimethylacetal (1,3 mmol) wurden in 4 ml n-Methyl-2-Pyrrolidon gelöst, und 22 mg wasserfreie p-Toluolsulfonsäure (0,13 mmol) wurden hinzugefügt. Der Reaktionsansatz wurde für drei Stunden auf 120 °C erwärmt, und das kondensierte Methanol wurde von dem Reaktionsansatz durch Kryodestillation entfernt. Nach Abkühlung auf Raumtemperatur wurde die Reaktion durch die Zugabe von 1 ml wässrigen Ammoniaks gequencht. Das n-Methyl-2-Pyrrolidon wurde durch Kryodestillation abgedampft, und der verbleibende Rest in basifiziertem Wasser erneut gelöst (basifiziertes Wasser enthält 0,05 Gew.-% wässrigen Ammoniak). Die Lösung wurde im basifizierten Wasser für zwei Stunden dialysiert, wobei das Dialysat alle drei Stunden gewechselt wurde. Nach Gefriertrocknung wurde mit sieben p-Propargyloxy-Benzacetal-Einheiten funktionalisiertes hPG$_{7,7}$ als viskoses Wachs erhalten. Die Umsetzung erfolgte zu 70 % bei einer Ausbeute von 80 %.

**Herstellung von Homobifunktionellem IPG$_5$-Biscyclooctin**

P(EEGE)$_5$-Br (4 g, 0.8 mmol) wurde in Tetrahydrofuran (20 ml) gelöst und die Lösung wurde mittels eines Eisbades auf 4 °C abgekühlt. Nach der Zugabe von Triethylamine (2.23 ml, 16 mmol) und Mesylchlorid (0.62 ml, 8 mmol) wurde die Reaktion bei Raumtemperatur für einen Tag durchgeführt. Nach Salzfiltration wurde das Polymer mittels Dialyse in THF aufgereinigt. Im Folgenden wurde das Polymer (2 g, 0.4 mmol) in DMF (20 ml) aufgenommen und mit NaN$_3$ (520 mg, 8 mmol) bei 80 °C drei Tage umgesetzt, das Salz abfiltriert, die Schutzgruppen mittels ethanolischer HCL (1 vol.%) entschützt und dann mittels dreitägiger Dialyse gereinigt. Danach wurde das azidierte Polymer (1.8 g, 0.36 mmol) in einer Wasser THF Mischung (10 ml, 1:1) durch Triphenylphosphin (377.3 mg, 1.44 mmol) 3 Tage reduziert. Das gebildete Diamin (1 g, 0.2 mmol) wurde letztlich mit BCN (138.6 mg, 0.44 mmol) in Dichlormethan (10 ml) mit Triethylamin (0.88 mmol, 123 µL) als Base. Das Polymer wurde mittels dreitägiger Dialyse in einer Wasser-Acetonmischung (1:1) aufgearbeitet um IPG$_5$-Biscyclooctin zu erhalten.

**Herstellung eines Polyglycerin-Nanogels durch Nanopräzipitation (Nanoausfällung)**

5 mg hPG$_{7,7}$-7-p-Propargyloxy-Benzacetal (0,6 µmol) und 7 mg hPG$_{7,7}$[N$_3$]$_7$ (0,9 µmol) wurden unabhängig voneinander in 0,5 ml gereinigtem deionisierten Wasser gelöst. Tris(3-Hydroxypropyltriazolylmethyl) (THPTA), Kupfersulfat und Natriumascorbat wurden in genau dieser Reihenfolge zu der hPG$_{7,7}$-7-p-Propargyloxy-Benzacetal-Lösung gegeben. Die Lösungen wurden auf 4 °C abgekühlt. Die Lösungen wurden dann miteinander gemischt und schnell in 20 ml Aceton, welches durch einen Magnetrührer gerührt wurde, gegeben. Nun kam es zur Ausfällung von Polyglycerin-Nanopartikeln, die als bläulich scheinende Dispersionen sichtbar waren. Die Partikelgröße wurde mittels dynamischer Lichtstreuung (DLS) ermittelt. Nach drei Stunden wurde die Gelbildungsreaktion durch die Zugabe eines Überschusses von 50 mg Azidoglycerol (427 µmol) gequencht. Nach 12 Stunden wurden 20 ml gereinigtes deionisiertes Wasser zugegeben, und das Aceton wurde abgedampft, um eine bläulich schimmernde Nanogel-Dispersion in Wasser zu erhalten. Das Nanogel wurde durch Zentrifugation bei 4.000 rpm von der wässrigen Phase abgetrennt und fünf Mal mit gereinigtem deionisierten Wasser gewaschen. Das Nanogel wurde anschließend mittels DLS, optischer Mikroskopie und Transmissionselektronenmikroskopie charakterisiert.

**Einbettung von Proteinen, unter anderem einer L-Asparaginase II, eines Rinderserumalbumins, des Antikörpers IgG und eines Lysozyms, in das Nanogel**

2 mg hPG$_{7,7}$-7-p-Propargyloxy-Benzacetal (0,2 µmol) und 3 mg hPG$_{7,7}$[N$_3$]$_7$ (0,3 µmol) wurden unabhängig voneinander in 0,5 ml gereinigtem deionisierten Wasser gelöst. THPTA und Kupferacetat wurden zu der hPG$_{7,7}$-7-p-Propargyloxy-Benzacetal-Lösung zugegeben. Ferner wurde das Protein zur hPG$_{7,7}$[N$_3$]$_7$-Lösung hinzugegeben. Die Lösungen wurden auf 4 °C abgekühlt. Anschließend wurden die Lösungen gemischt und schnell zu 20 ml Aceton gegeben, welches auf einem Magnetrührer gerührt wurde. Nach drei Stunden wurde die Gelbildungsreaktion durch die Zugabe eines Überschusses von 50 mg Azidoglycerol (427 µmol) gequencht. Nach 12 Stunden wurde das Nanogel mittels Zentrifugation bei 4.000 rpm von der flüssigen Phase abgetrennt und fünf Mal mit gereinigtem deionisierten Wasser gewaschen.

**Einbettung von Proteinen, unter anderem einer L-Asparaginase II, eines Rinderserumalbumins, des Antikörpers IgG und eines Lysozyms, in das Nanogel durch kupferfreie Klick-Chemie**

2 mg hPG$_{7,7}$-7-p-Azidopropoxy-Benzacetal (0,2 µmol) und 4 mg IPG$_5$-Biscyclooctin (0,6 µmol) wurden unabhängig voneinander in 0,5 ml gereinigtem deionisierten Wasser gelöst. Ferner wurde das Protein zur hPG$_{7,7}$-7-p-Azidopropoxy-Benzacetal Lösung hinzugegeben. Die Lösungen wurden auf 4 °C abgekühlt. Anschließend wurden die Lösungen gemischt und schnell zu 20 ml Aceton gegeben, welches auf einem Magnetrührer gerührt wurde. Nach drei Stunden wurde die Gelbildungsreaktion durch die Zugabe eines Überschusses von 50 mg Azidoglycerol (427 µmol) gequencht. Nach 12 Stunden wurde das Nanogel mittels Zentrifugation bei 4.000 rpm von der flüssigen Phase abgetrennt und fünf Mal mit gereinigtem deionisierten Wasser gewaschen.

**Größenbestimmung der Nanogel-Partikel**

Wie die nachfolgende Tabelle 1 zeigt, hängt die Größe der erhaltenen Polyglycerin-Nanogele von der Ausgangskonzentration der eingesetzten Makromonomere ab.

**Tabelle 1: Abhängigkeit der Größe der gebildeten Polyglycerin-Nanopartikel von der Ausgangskonzentration der eingesetzten Makromonomere.**

| C (Makromonomer) / (mg/ml) | d/nm (in Aceton) | PDI (in Aceton) | d/nm (in Wasser) | PDI (in Wasser) |
|---|---|---|---|---|
| 12 | 580 | 0,03 | 820 | 0,07 |
| 6 | 440 | 0,02 | 610 | 0,03 |
| 3 | 310 | 0,06 | 430 | 0,08 |
| 1.5 | 102 | 0,04 | 145 | 0,07 |

Je geringer die Ausgangskonzentration der Makromonomere ist, desto kleiner ist der Durchmesser der gebildeten Nanogele. In der Tabelle 1 bezeichnet dabei c die Konzentration, d den Durchmesser und PDI die Polydispersität. Während bei einer Makromonomerkonzentration von 1,5 mg/ml Polyglycerin-Nanogele mit einem Durchmesser von rund 100 nm in Aceton erhalten wurden, stieg dieser Durchmesser bei einer Ausgangskonzentration von 12 mg/ml Makromonomeren auf knapp 600 nm an. Nach Überführung der Nanogele in Wasser kam es zum weiteren Quellen der Nanogele durch Einbau von Wassermolekülen. Dadurch vergrößerte sich auch der gemessene Durchmesser der Nanogele.

Die Polydispersität ist ein Maß für die Streuung der Partikelgrößen und weist darauf hin, dass die Nanogele eine sehr enge Größenverteilung haben. Werden die Partikel von Aceton in Wasser überführt steigen die Partikelgrößen an. Dies deutet auf das Anquellen der Partikel hin.

**Bestimmung der Nanogel-Degradationskinetik**

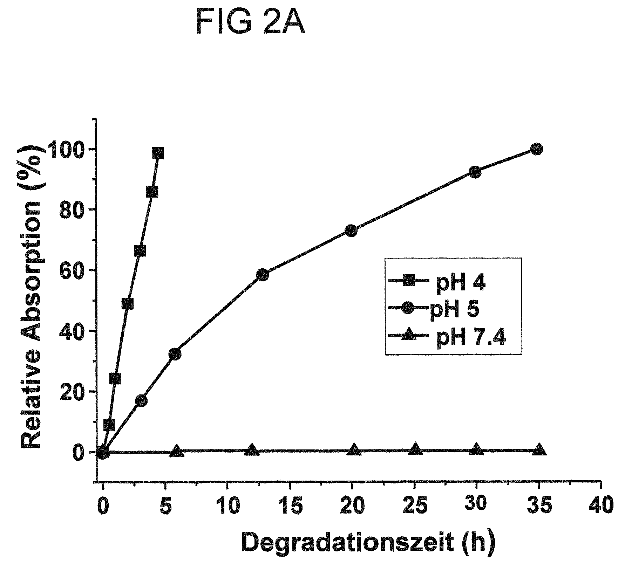

Nanogel-Dispersionen wurden bei 37 °C und bei unterschiedlichen pH-Werten inkubiert. Nach unterschiedlichen Inkubationszeiten wurden die Nanogele auf 4 °C abgekühlt, neutralisiert und von degradierten Fragmenten mittels einer 5-minütigen Zentrifugation bei 4.000 rpm abgetrennt. Anschließend wurde die UV-Absorption der abgebauten Fragmente, welche sich in der überstehenden Lösung befinden, bei 350 nm beobachtet. Während des Abbaus gehen immer mehr abgebaute Fragmente in Lösung wodurch die Absorption ansteigt. Das entsprechende Ergebnis dieses Versuches ist in der Figur 2A dargestellt. Gut zu erkennen ist, dass bei einem pH-Wert von 7,4 die Integrität der Polyglycerin-Nanopartikel nicht angegriffen wird. Vielmehr bleiben sie bei diesem pH-Wert stabil. Erst wenn der pH-Wert abgesenkt wird, kommt es zu einer Degradation der Polyglycerin-Nanopartikel, da dann die in den gebildeten Nanopartikeln enthaltene Benzacetalbindung aufgebrochen wird. Wie aus der Figur 2A ersichtlich ist, verläuft die Degradation der mit Benzacetalgruppen versehenen Polyglycerin-Nanopartikeln umso schneller, je niedriger der eingestellte pH-Wert ist. Bei einem pH-Wert von 4 sind die Polyglycerin-Nanopartikel in weniger als fünf Stunden vollständig abgebaut.

Die vollständige Nanogel-Degradation wurde mittels DLS-Größenmessungen und $^1$H-NMR-spektroskopischen Messungen bestätigt.

**Durch pH-abhängige Nanogel-Degradation ausgelöste kontrollierte Freisetzung von Asparaginase**

Das mit L-Asparaginase II gemäß dem oben erläuterten Protokoll beladene Polyglycerin-Nanogel (10 mg/ml Nanogel und 0,5 mg/ml L-Asparaginase II) wurde mit Salzsäure auf pH 4 bzw. pH 5 angesäuert. Die Proben wurden bei Raumtemperatur (25 $\pm$ 2 °C) unter leichter Bewegung (300 rpm) inkubiert. Einzelne Proben wurden über 3 Tagen gesammelt und anschließend mittels Größenausschluss-Hochleistungschromatographie (SEC-HPLC) analysiert. Um die Nanogel-Degradation zu stoppen, wurden die Proben vor der SEC-HPLC mit 0,1 M Kalilauge neutralisiert. Für die SEC-HPLC wurden 50 $\mu$l der neutralisierten Proben in eine HPLC injiziert, das mit einer TSKgel G40000 PWXL-Säule (300 x 7,8 mm, 10 $\mu$m Partikelgröße) ausgestattet war. Es erfolgte eine isokratische Elution mit einem Puffer aus 20 mM NaHPO4, 150 mM NaCl und 0,003 mM NaN3 (pH 7,4) bei einer Flussrate von 0,4 ml/min. Die Konzentration der L-Asparaginase II wurde mittels UV-Absorption bei 280 nm und Fluoreszenzdetektion (Anregung mit 295 nm und Emission bei 348 nm) bestimmt.

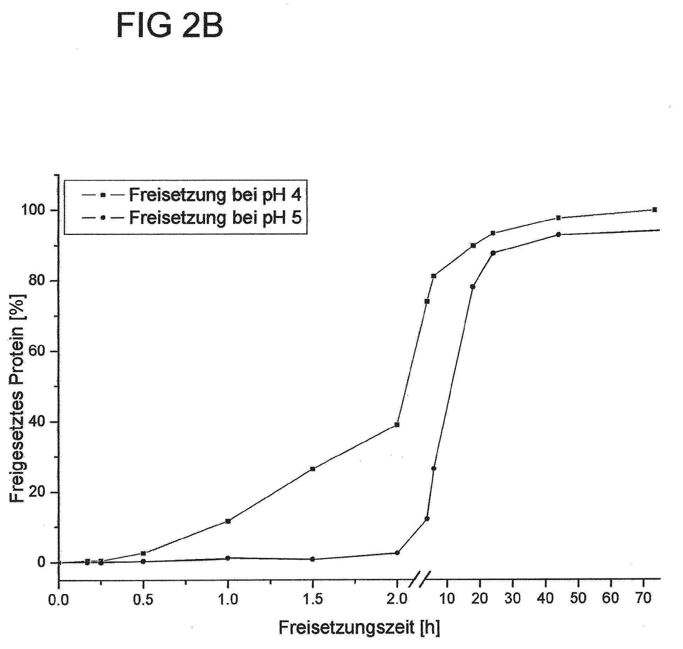

Die Figur 2B zeigt die entsprechenden Ergebnisse dieser Untersuchung. Die erhaltenen HPLC-Chromatogramme zeigen, dass L-Asparaginase II mittels der intrinsischen Tryptophanfluoreszenz ohne Beeinträchtigung durch Polyglycerin oder Polyglycerinfragmente detektiert werden kann. Beim intakten mit L-Asparaginase II beladenen Polyglycerin-Nanogel konnte keine freie L-Asparaginase detektiert werden. Die Beladungs- oder Einbettungseffizienz lag folglich bei annähernd 100 %. Mittels der Chromatogramme wurde der Anteil der freigesetzten L-Asparaginase II bestimmt und in der Figur 2B über die Zeit aufgetragen. Erwartungsgemäß wurde die L-Asparaginase II bei pH 4 (quadratische Datenpunkte) schneller freigesetzt als bei pH 5 (runde Datenpunkte), da beim niedrigeren pH-Wert eine schnellere Nanogel-Degradation erfolgt.

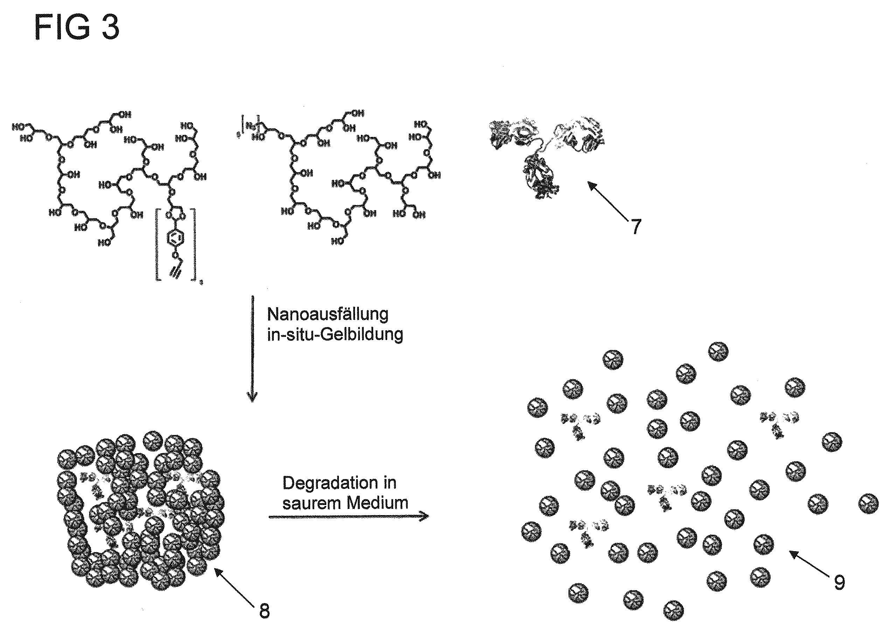

Die Figur 3 zeigt eine schematische Darstellung der zuvor erläuterten Einbettung eines Proteins 7 in die gebildeten Nanopartikel bzw. das gebildete Nanogel 6. Durch eine gemeinsame Nanoausfällung von Polyglycerin-Makromonomeren und einem Protein kommt es zu einer in-situ-Gelbildung, wobei die mit ausgefällten Proteine im Inneren des Nanogels in nativer Form eingebettet werden. Wird das proteinbeladene Polyglycerin-Nanogel 8 in ein saures Medium überführt, kommt es zur Degradation und zur Proteinfreisetzung 9. Diesen Umstand kann man sich zu Nutze machen, da in entzündeten bzw. Tumorgewebe eines Organismus niedrige pH-Werte vorherrschen. Auf diese Weise ist es möglich, ein mit einem therapeutischen Protein beladenes Nanogel in einen Organismus einzuschleusen, wobei das therapeutische Protein erst am Ort seiner Wirkung (nämlich dem entzündeten Gewebe mit niedrigem pH-Wert) freigesetzt wird. Statt Einbettung oder Einbindung kann auch von Verkapselung eines Proteins bzw. eines anderen aktiven Wirkstoffs gesprochen werden.

Wie bereits erwähnt, sind harsche Reaktionsbedingungen nicht dafür geeignet, ein Protein bzw. eine andere labile Substanz in ihrer nativen und aktiven Form zu erhalten. Um zu beweisen, dass mit dem vorliegend vorgestellten Verfahren Proteine in nativer Form in das gebildete Nanogel eingebettet werden können, wurde die Sekundärstruktur von L-Asparaginase II als beispielhaft eingebettetes Enzym nach Verkapslung in und Freisetzung aus den Nanogelen ermittelt. Dies erfolgte mittels der Fourier-Transformations-Infrarotspektroskopie (FTIR), wobei in Form abgeschwächter Totalreflexions-Messungen (ATR) gemessen wurde. Die eingesetzte ATR-Zelle wurde bei einer konstanten Temperatur von 25 °C gehalten. 25 $\mu$l einer Probe wurden unter trockenem Stickstoff auf die ATR-Zelle gegeben und gegen PBS-Puffer mit einem pH-Wert von 5 oder gegen Wasser als Kontrolle gemessen. Es wurden 120 Scans für jedes Experiment bei einer Auflösung von 4 cm$^{-1}$ durchgeführt, wobei eine Wasserdampfkorrektur erfolgte. Die zweiten Ableitungen der erhaltenen Absorptionsspektren wurden für die weitere Datenanalyse verwendet.

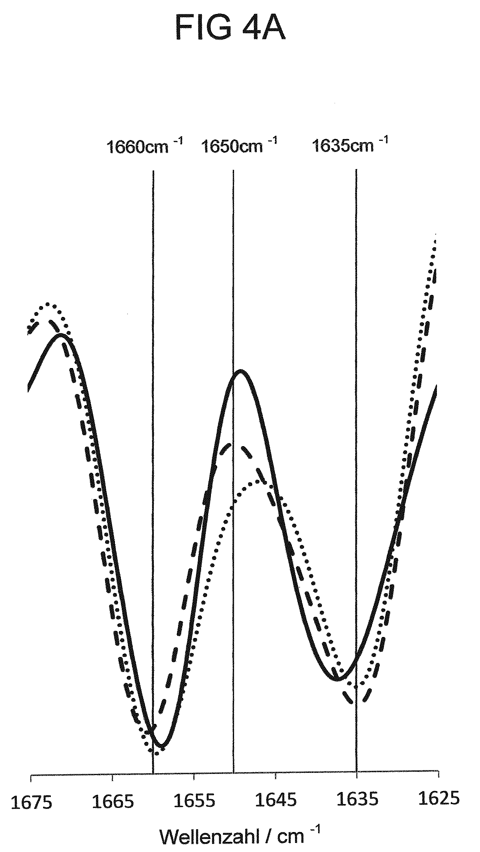

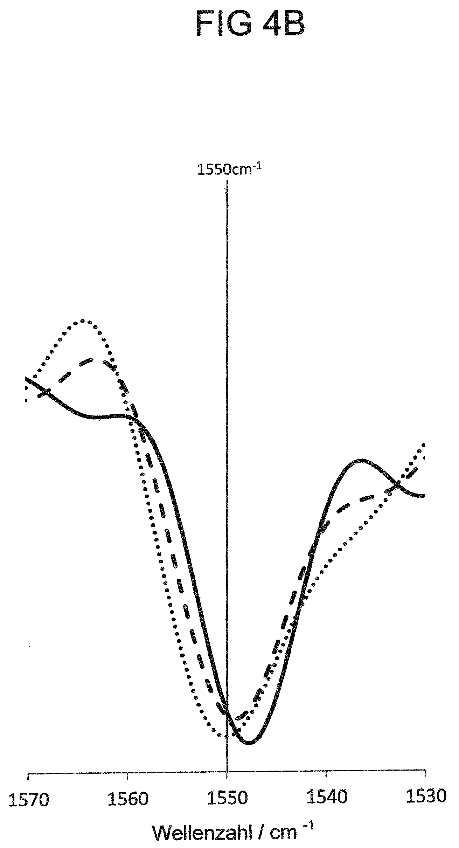

Das Ergebnis dieser FTIR-Untersuchungen ist in den Figuren 4A und 4B dargestellt. Dabei zeigt die Figur 4A Absorptionen, die im Bereich der Amid-I-Bande beobachtet wurden, während in der Figur 4B Absorptionen im Bereich der Amid-II-Bande dargestellt sind. Die Amid-I-Bande ist sensitiv für C=O-Streckschwingungen und eignet sich gut zur Bestimmung der Sekundärstruktur eines Proteins.

Die zweite Ableitung des Spektrums nativer, in Wasser gelöster L-Asparaginase II (frisch zubereitet) ist als gestrichelte Linie in den Figuren 4A und 4B dargestellt. Die gepunktete Linie zeigt die zweite Ableitung eines Absorptionsspektrums von L-Asparaginase II, die sieben Tage in PBS-Puffer bei einem pH-Wert von 5 gelagert wurde. Die durchgehende Linie zeigt schließlich die zweite Ableitung eines Absorptionsspektrums von L-Asparaginase II, die nach sieben Tagen aus Polyglycerin-Nanogelen freigesetzt wurde.

Wie aus der Figur 4A zu sehen ist, unterscheiden sich weder die Bandenintensitäten noch die Wellenzahlen der drei verschiedenen Proben signifikant voneinander. Vielmehr kann lediglich eine leichte Verschiebung der für eine $\alpha$-helikale Sekundärstruktur charakteristischen Bande bei 1660 cm$^{-1}$ um 1 cm$^{-1}$ zu niedrigeren Wellenzahlen beobachtet werden. Bei der für eine $\beta$-Faltblatt-Sekundärstruktur charakteristischen Bande bei etwa 1635 cm$^{-1}$ für das native Protein kann keine Verschiebung beobachtet werden. Insgesamt liegen diese Verschiebungen jedoch innerhalb der messtechnisch bedingten Fehlerbreite. Somit ist davon auszugehen , dass sich die Sekundärstruktur durch eine Einkapselung der L-Asparaginase II in die Polyglycerin-Nanopartikel nicht ändert.

Dieser Befund wird auch durch eine Analyse der Amid-II-Bande bestätigt. Die Amid-II-Bande liefert Informationen über die N-H-Biegeschwingungen und die C-N-Streckschwingungen. Hier kann durch eine Lagerung der L-Asparaginase II in Wasser bzw. eine Verkapselung dieses Enzyms in den Polyglycerin-Nanogelen ebenfalls keine signifikante Bandenverschiebung (siehe Figur 4B)festgestellt werden.

Die beobachteten Absorptionen im Amid-I- und Amid-II-Bereich sind in der nachfolgenden Tabelle 2 dargestellt.

**Tabelle 2: Absorptionen der L-Asparaginase im Amid-I- und Amid-II-Bereich, bestimmt anhand der zweiten Ableitungen entsprechender Absorptionsspektren.**

| | Absorptionen im Bereich der Amid-I-Bande / cm$^{-1}$ | | Absorptionen im Bereich der Amid-II-Bande / cm$^{-1}$ |
|---|---|---|---|
| L-Asparaginase II in Wasser (frisch zubereitet) | 1660,5 | 1634,4 | 1549,6 |
| L-Asparaginase II in PBS pH 5.0 (7d Lagerung) | 1659,5 | 1635,4 | 1550,5 |
| L-Asparaginase II nach Freisetzung (7d Lagerung) | 1659,0 | 1637,3 | 1547,6 |

**Bestimmung der Asparaginase-Aktivität**

Die Aktivität der L-Asparaginase II wurde mittels Neßlers Ammoniak-Quantifizierung durchgeführt.

Für die Durchführung des Asparaginase-Aktivitätstests wurden 50 μl L-Asparaginase II, 100 μl Tris-HCl mit einem pH-Wert von 8,6 und 850 μl L-Asparagin-Monohydrat-Pufferlösung bei 37 °C für 10 Minuten inkubiert. Nach Zugabe von 50 μl einer 1,5 M Lösung von Trichloressigsäure und anschließender Zentrifugation wurden 100 μl des Überstandes zu Neßlers Reagenz gegeben. Nach 10 Minuten wurde die optische Dichte bei 436 nm bestimmt und mit einer Kalibrierungskurve verglichen sowie um den Gesamtenzymgehalt korrigiert. Die Berechnung der Enzymaktivität erfolgte dann nach folgender Formel:

$$U/mg = \frac{\mu mol\ freigesetzten\ Ammoniaks}{10\ min \times mg\ Enzym\ in\ der\ \text{Re}\ aktion}$$

Eine Einheit (1 U) der ermittelten Enzymaktivität entsprechen dabei der freigesetzten Menge an Ammoniak in Mikromol pro 10 Minuten aus Asparagin als Substrat.

Diese Quantifizierung ergab eine Aktivität der frisch zubereiteten Asparaginase-Lösung von 98,6 U/mg, was sich mit den vom Hersteller angegebenen Daten (98,2 U/mg) deckte. Wird L-Asparaginase II in einen PBS-Puffer mit pH 5,0 überführt verringerte sich die Aktivität um 10 % auf 86,1 U/mg. Die Lagerung der L-Asparaginase II in dem Puffer über 7 Tage reduziert die Aktivität um weitere 2,5 % auf 86,2 U/mg. Ein im Rahmen der Messgenauigkeit identischer Wert konnte für die Aktivität der in ein Polyglycerin-Nanogel eingekapselten und wieder freigesetzten L-Asparaginase II nach deren Freisetzung ermittelt werden. Die Messergebnisse sind in der nachfolgenden Tabelle 3 zusammen mit den jeweils ermittelten Standardabweichungen (SD) dargestellt.

**Tabelle 3: Enzymaktivität der L-Asparaginase II.**

| | Spezifische Aktivität / (U/mg) | Spezifische Aktivität / % | SD / (U/mg) | SD / % |
|---|---|---|---|---|
| L-Asparaginase II in Wasser (frisch zubereitet) | 98,6 | 100 | 4,4 | 4,4 |
| L-Asparaginase II in PBS pH 5.0 (frisch zubereitet) | 89,1 | 90,8 | 0,1 | 0,1 |
| L-Asparaginase II in PBS pH 5.0 (7d Lagerung) | 86,1 | 87,3 | 0,7 | 0,8 |
| L-Asparaginase II nach Freisetzung (7d Lagerung) | 86,2 | 87,5 | 0,9 | 1 |

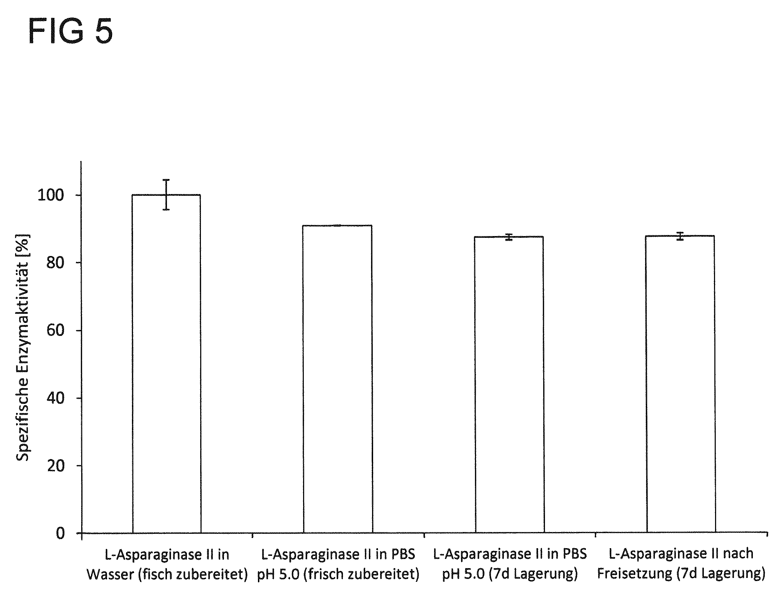

Eine entsprechende grafische Darstellung der spezifischen Enzymaktivität ist der Figur 5 zu entnehmen. Dabei wurde die Enzymaktivität der frisch zubereiteten nativen L-Asparaginase II in Wasser auf 100 % gesetzt. Auch aus dieser grafischen Darstellung ist ersichtlich, dass die in ein Polyglycerin-Nanogel eingekapselte L-Asparaginase II nach ihrer Freisetzung eine Aktivität aufweist, die der Aktivität von in Puffer gelöster L-Asparaginase II entspricht. Dies bestätigt die durch die FTIR-Messungen ermittelten Resultate. So behält L-Asparaginase II auch nach einer Einkapselung in ein Polyglycerin-Nanogel ihre nativeSekundärstruktur. Ferner reduziert eine Verkapselung von L-Asparaginase II in Polyglycerin-Nanogelen nicht die Enzymaktivität.

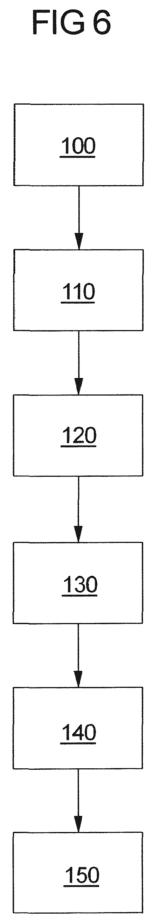

Die Figur 6 zeigt ein Flussdiagramm, das der Veranschaulichung eines Ausführungsbeispiels des beanspruchten Verfahrens dient.

In einem dem eigentlichen Verfahren vorgelagerten Schritt 100 erfolgt eine Synthese erster Polyglycerin-Makromonomere und zweiter Polyglycerin-Makromonomere.

In einem ersten Verfahrensschritt 110 erfolgt eine aktive Vereinigung der ersten und der zweiten Polyglycerin-Makromonomere, einer labilen Substanz (beispielsweise ein Protein oder Enzym) und bei Bedarf eines Katalysators, der die später erfolgende Klick-Reaktion katalysiert.

In einem zweiten Verfahrensschritt 120 erfolgt eine aktive Überführung der vereinigten Substanzen in ein organisches Nichtlösungsmittel. "Aktiv" bedeutet dabei, dass ein Operateur die entsprechenden Schritte durch aktive Handlungen ausführt.

In einem dritten Verfahrensschritt 130 kommt es zu zwei spontanen chemischen Reaktionen, so dass man auch von Doppelspontanität sprechen kann. Einerseits erfolgt ein spontanes Ausfällen der vereinigten Substanzen unter spontaner Bildung von Nano-Aggregaten. Andererseits erfolgt eine spontane Vernetzung der Nano-Aggregate durch eine Klick-Reaktion (unter Ausbildung kovalenter Bindungen zwischen den ersten Polyglycerin-Makromonomeren und den zweiten Polyglycerin-Makromonomeren).

Danach werden die vernetzten Nanopartikel in einem vierten Verfahrensschritt 140 aktiv in eine wässrige Phase überführt.

Anschließend erfolgt in einem fünften Verfahrensschritt 150 ein spontanes Quellen der vernetzten Partikel in wässriger Phase.

## Patentansprüche

1.  Verfahren zur Herstellung eines Polyglycerin-Nanogels, umfassend die folgenden Schritte:

    - Mischen einer wässrigen Lösung von ersten Polyglycerin-Makromonomeren (1), die mit einer ersten reaktiven Gruppe (10) modifiziert sind, mit einer wässrigen Lösung von zweiten Polyglycerin-Makromonomeren (2), die mit einer zweiten reaktiven Gruppe (20) modifiziert sind, wobei die erste reaktive Gruppe (10) und die zweite reaktive Gruppe (20) unter Ausbildung einer kovalenten Bindung miteinander reagieren können,
    - Überführen der Mischung in ein organisches Nichtlösungsmittel (4),
    - Ausfällen eines Polyglycerin-Nanogels (6), das aus miteinander kovalent verbundenen ersten Polyglycerin-Makromonomeren (1) und zweiten Polyglycerin-Makromonomeren (2) besteht, wobei die kovalente Bindung zwischen den ersten Polyglycerin-Makromonomeren (1) und den zweiten Polyglycerin-Makromonomeren (2) durch eine Reaktion der ersten reaktiven Gruppe (10) und der zweiten reaktiven Gruppe (20) hergestellt wird, die infolge der Überführung der Mischung in das organische Nichtlösungsmittel (4) abläuft,

    **dadurch gekennzeichnet,**
    **dass** das organische Nichtlösungsmittel (4) mit Wasser (3) mischbar ist und dass das Verfahren ohne Zugabe einer oberflächenaktiven Substanz durchgeführt wird.

2.  Verfahren nach Anspruch 1, **dadurch gekennzeichnet, dass** das Verfahren ohne Einsatz von Ultraschall durchgeführt wird.

3.  Verfahren nach Anspruch 1 oder 2, **dadurch gekennzeichnet, dass** das Ausfällen des Polyglycerin-Nanogels (6) nach Überführen der Mischung in das organische Lösungsmittel spontan abläuft.

4.  Verfahren nach einem der vorhergehenden Ansprüche, **dadurch gekennzeichnet, dass** die ersten Polyglycerin-Makromonomere (1) in einer ersten Konzentration und die zweiten Polyglycerin-Makromonomere (2) in einer zweiten Konzentration vorliegen, wobei die erste und die zweite Konzentration unabhängig voneinander im Bereich von 0,1 bis 30 mg/ml liegen.

5. Verfahren nach einem der vorhergehenden Ansprüche, **dadurch gekennzeichnet, dass** das Polyglycerin-Nanogel (6) nach dem Ausfällen in eine wässrige Phase überführt wird.

6. Verfahren nach einem der vorhergehenden Ansprüche, **dadurch gekennzeichnet, dass** das Ausfällen in Anwesenheit einer labilen Substanz wie zum Beispiel eines Peptids, eines Proteins (7), DNA, RNA und/oder eines Hormons und/oder in Anwesenheit einer biologisch aktiven Substanz durchgeführt wird.

7. Verfahren nach einem der vorhergehenden Ansprüche, **dadurch gekennzeichnet, dass** die ersten Polyglycerin-Makromonomere (1) und/oder die zweiten Polyglycerin-Makromonomere (2) eine pH-labile Gruppe enthalten, die im gebildeten Polyglycerin-Nanogel (6) noch vorhanden ist.

8. Verfahren nach Anspruch 7, **dadurch gekennzeichnet, dass** die pH-labile Gruppe ausgewählt ist aus der Gruppe bestehend aus Acetalen, Ketalen, Enolethern, Estern, Amiden Hydrazonen, Hydraziden, Oximen, Maleinsäurederivaten, Carbamaten, Hydroxylaminen Iminen, Imminiumverbindungen, Enaminen, Silylethern und Silylenolethern.

9. Verfahren nach einem der vorhergehenden Ansprüche, **dadurch gekennzeichnet, dass** die ersten Polyglycerin-Makromonomere (1) und/oder die zweiten Polyglycerin-Makromonomere (2) eine endständige Modifizierung des Typs -R-R' aufweisen, die kovalent mit einem linearen oder verzweigten Polyglyceringerüst der ersten Polyglycerin-Makromonomere (1) und/oder der zweiten Polyglycerin-Makromonomere (2)verbunden ist, wobei R eine pH-labile Gruppe ist und R' eine bioorthogonale endständige Gruppe ist, die eine Reaktion gemäß der Klick-Chemie eingehen kann.

10. Verfahren nach einem der vorhergehenden Ansprüche, **dadurch gekennzeichnet, dass** es bei einer Temperatur von 0 °C bis 25 °C durchgeführt wird.

11. Verfahren nach einem der vorhergehenden Ansprüche, **dadurch gekennzeichnet, dass** die erste reaktive Gruppe (10) eine Alkingruppe und die zweite reaktive Gruppe eine Azidgruppe (20) ist.

12. Verfahren nach einem der vorhergehenden Ansprüche, **dadurch gekennzeichnet, dass** das Ausfällen des Polyglycerin-Nanogels ohne Zugabe einer kupferhaltigen Verbindung erfolgt.


**Claims**

1. Method for producing a polyglycerol nanogel, comprising the following steps:

    - mixing an aqueous solution of first polyglycerol macromonomers (1), which are modified with a first reactive group (10), with an aqueous solution of second polyglycerol macromonomers (2), which are modified with a second reactive group (20), wherein the first reactive group (10) and the second reactive group (20) can react with each other forming a covalent bond,
    - transferring the mixture into an organic non-solvent (4),
    - precipitation of a polyglycerol nanogel (6) consisting of first polyglycerol macromonomers (1) and second polyglycerol macromonomers (2) which are covalently bound to each other, wherein the covalent bond between the first polyglycerol macromonomers (1) and the second polyglycerol macromonomers (2) is established by a reaction of the first reactive group (10) and the second reactive group (20), which takes place as a consequence of transferring the mixture into the organic non-solvent (4),

    **characterized**
    **in that** the organic non-solvent (4) is miscible with water (3) and in that the method is carried out without adding a surface-active substance.

2. Method according to Claim 1, **characterized in that** the method is carried out without using ultrasound.

3. Method according to Claim 1 or 2, **characterized in that** the precipitation of the polyglycerol nanogel (6) takes place spontaneously after transferring the mixture into the organic solvent.

4. Method according to any of the preceding claims, **characterized in that** the first polyglycerol macromonomers (1) are present in a first concentration and the second polyglycerol macromonomers (2) are present in a second concentration, wherein the first and the second concentration lie in a range of 0.1 to 30 mg/ml independently of each other.

5. Method according to any of the preceding claims, **characterized in that** the polyglycerol nanogel (6) is transferred into an aqueous phase after precipitation.

6. Method according to any of the preceding claims, **characterized in that** the precipitation is carried out in the presence of a labile substance such as, for instance, a peptide, a protein (7), DNA, RNA and/or a hormone and/or in the presence of a biologically active substance.

7. Method according to any of the preceding claims, **characterized in that** the first polyglycerol macromonomers (1) and/or the second polyglycerol macromonomers (2) contain a pH-labile group, which is still present in the polyglycerol nanogel (6) formed.

8. Method according to Claim 7, **characterized in that** the pH-labile group is selected from the group consisting of acetals, ketals, enol ethers, esters, amides, hydrazones, hydrazides, oximes, maleic acid derivatives, carbamates, hydroxylamine imines, iminium compounds, enamines, silyl ethers and silyl enol ethers.

9. Method according to any of the preceding claims, **characterized in that** the first polyglycerol macromonomers (1) and/or the second polyglycerol macromonomers (2) have a terminal modification of the type -R-R', which is covalently bound to a linear or branched polyglycerol structure of the first polyglycerol macromonomers (1) and/or of the second polyglycerol macromonomers (2), wherein R is a pH-labile group and R' is a bioorthogonal terminal group that can undergo a reaction according to click chemistry.

10. Method according to any of the preceding claims, **characterized in that** it is carried out at a temperature of 0 °C to 25 °C.

11. Method according to any of the preceding claims, **characterized in that** the first reactive group (10) is an alkyne group and the second reactive group is an azide group (20).

12. Method according to any of the preceding claims, **characterized in that** the precipitation of the polyglycerol nanogel takes place without adding a compound containing copper.

**Revendications**

1. Procédé pour fabriquer un nanogel de polyglycérol, comprenant les étapes suivantes :

   - le mélange d'une solution aqueuse de premiers macromonomères de polyglycérol (1), qui sont modifiés par un premier groupe (10) réactif, avec une solution aqueuse de deuxièmes macromonomères de polyglycérol (2), qui sont modifiés par un deuxième groupe (20) réactif, dans lequel le premier groupe (10) réactif et le deuxième groupe (20) réactif peuvent réagir l'un avec l'autre en formant une liaison covalente,
   - la transformation du mélange en un non-solvant (4) organique,
   - la précipitation d'un nanogel de polyglycérol (6), qui est constitué de premiers macromonomères de polyglycérol (1) et de deuxièmes macromonomères de polyglycérol (2) reliés les uns aux autres de manière covalente, dans lequel la liaison covalente entre les premiers macromonomères de polyglycérol (1) et les deuxièmes macromonomères de polyglycérol (2) est établie par une réaction du premier groupe (10) réactif et du deuxième groupe (20) réactif, laquelle se déroule suite à la transformation du mélange en le non-solvant (4) organique,

   **caractérisé en ce**
   **que** le non-solvant (4) organique est miscible avec l'eau (3), et que le procédé est réalisé sans l'ajout d'une substance tensioactive.

2. Procédé selon la revendication 1, **caractérisé en ce que** le procédé est réalisé sans l'utilisation d'ultrasons.

3. Procédé selon la revendication 1 ou 2, **caractérisé en ce que** la précipitation du nanogel de polyglycérol (6) se déroule de manière spontanée après la transformation du mélange en le solvant organique.

4. Procédé selon l'une quelconque des revendications précédentes, **caractérisé en ce que** les premiers macromonomères de polyglycérol (1) sont présents en une première concentration et les deuxièmes macromonomères de polyglycérol (2) sont présents en une deuxième concentration, dans lequel la première et la deuxième concentration se situent indépendamment l'une de l'autre dans la plage allant de 0,1 à 30 mg/ml.

5. Procédé selon l'une quelconque des revendications précédentes, **caractérisé en ce que** le nanogel de polyglycérol (6) est transformé, après la précipitation, en une phase aqueuse.

6. Procédé selon l'une quelconque des revendications précédentes, **caractérisé en ce que** la précipitation est réalisée en présence d'une substance labile telle que par exemple un peptide, une protéine (7), de l'ADN, de l'ARN et/ou une hormone et/ou en présence d'une substance biologiquement active.

7. Procédé selon l'une quelconque des revendications précédentes, **caractérisé en ce que** les premiers macromonomères de polyglycérol (1) et/ou les deuxièmes macromonomères de polyglycérol (2) contiennent un groupe à pH labile, qui est encore présent dans le nanogel de polyglycérol (6) obtenu.

8. Procédé selon la revendication 7, **caractérisé en ce que** le groupe à pH labile est choisi parmi le groupe comprenant des acétals, des cétals, des éthers énoliques, des esters, des amides, des hydrazones, des hydrazides, des oximes, des dérivés d'acide maléique, des carbamates, des hydroxylamines, des imines, des composés d'imminium, des énamines, des éthers de silyle et des éthers de silylénol.

9. Procédé selon l'une quelconque des revendications précédentes, **caractérisé en ce que** les premiers macromonomères de polyglycérol (1) et/ou les deuxièmes macromonomères de polyglycérol (2) présentent une modification terminale du type -R-R', qui est reliée de manière covalente à un squelette de polyglycérol linéaire ou ramifié des premiers macromonomères de polyglycérol (1) et/ou des deuxièmes macromonomères de polyglycérol (2), dans lequel R est un groupe à pH labile et R' est un groupe terminal bioorthogonal, qui peut entrer en réaction par chimie click.

10. Procédé selon l'une quelconque des revendications précédentes, **caractérisé en ce qu'**il est réalisé à une température allant de 0 °C à 25 °C.

11. Procédé selon l'une quelconque des revendications précédentes, **caractérisé en ce que** le premier groupe (10) réactif est un groupe alcyne et le deuxième groupe réactif est un groupe azide (20).

12. Procédé selon l'une quelconque des revendications précédentes, **caractérisé en ce que** la précipitation du nanogel de polyglycérol a lieu sans ajout d'un composé contenant du cuivre.

FIG 2A

Relative Absorption (%)

Degradationszeit (h)

pH 4
pH 5
pH 7.4

# FIG 2B

# FIG 3

Nanoausfällung
in-situ-Gelbildung

Degradation in
saurem Medium

EP 2 892 642 B1

# FIG 4A

1660cm⁻¹  1650cm⁻¹  1635cm⁻¹

1675   1665   1655   1645   1635   1625

Wellenzahl / cm⁻¹

# FIG 4B

## FIG 5

# FIG 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2138527 A1
- US 0090011420 A1

- DE 102008030992 A1


**In der Beschreibung aufgeführte Nicht-Patentliteratur**

- **A. L. SISSON ; I. PAPP ; K. LANDFESTER ; R. HAAG.** *Macromolecules,* 2009, vol. 42, 556-559
- **A. L. SISSON ; D. STEINHILBER ; T. ROSSOW ; P. WELKER ; K. LICHA ; R. HAAG.** *Ang. Chem. Int. Ed.,* 2009, vol. 48, 7540-7545
- **D. STEINHILBER ; A. L. SISSON ; D. MANGOLDT ; P. WELKER ; K. LICHA ; R. HAAG.** *Adv. Funct. Mater.,* 2010, vol. 20, 4133-4138
- **H. ZHOU ; D. STEINHILBER ; H. SCHLAAD ; A. L. SISSON ; R. HAAG.** *React. Funct, Polym.,* 2011, vol. 71, 356-361

- **PATTISON, DAVID I. ; ALDWIN SURYO RAHMANTO ; MICHAEL J. DAVIES.** Photo-oxidation of proteins. *Photochemical & Photobiological Sciences,* 2012, vol. 11 (1), 38-53
- **C. BERTOZZI et al.** *Angew. Chem. Int. Ed. Engl.,* 2009, vol. 48, 6974-6998
- **VON DICKERSON et al.** Prinzipien der Chemie. Walter de Gruyter Verlag, 1988, 187
- **K. B. SHARPLESS et al.** *Angew. Chem. Int. Ed. Engl.,* 2001, vol. 40, 2004-2021